# EUROPEAN PATENT APPLICATION

(11) **EP 4 035 660 A1**
(43) Date of publication of application: **03.08.2022**
(21) Application number: 22158492.3
(22) Date of filing: 17.10.2018
(51) Int. Cl.: A61K 9/24, A61K 31/18, A61K 31/4725

(54) **TABLETS COMPRISING TAMSULOSIN AND SOLIFENACIN**

(30) Priority: 17.10.2017 EP 17196922
(62) Divisional of application: 18785395.7
(71) Applicant: Synthon B.V., 6545 CM Nijmegen (NL)
(72) Inventor: KUMAR, Rohit, 08830 SantBoi de Llobregat (ES); ALVAREZ FERNADEZ, Lisardo, 08830 SantBoi de Llobregat (ES); GAGO GUILLAN, Manuel, 08830 SantBoi de Llobregat (ES); VELADA CALZADA, Jose, 6545 CM Nijmegen (NL)
(74) Representative: Tejedor Vinent, Henar

(57) **Abstract**

The present invention relates to a pharmaceutical multi layer tablet comprising a controlled release part with tamsulosin and an immediate release part wherein the immediate release formulation comprises: solifenacin succinate and a water insoluble diluent in an amount of 50 to 99% w/w relative to the total weight of the immediate release part of the tablet.

## Description

### BACKGROUND OF THE PRESENT INVENTION

Tamsulosin is a known chemical compound having alpha-adrenergic blocking activity that is useful for treatment of cardiac insufficiencies and benign prostatic hyperplasia (BPH). Tamsulosin is the common name for (R)-5-[ 2-[[2-(2-ethoxyphenoxy)ethyl]amino] propyl]-2-methoxy-benzenesulfonamide.

It has the structure of formula (I):

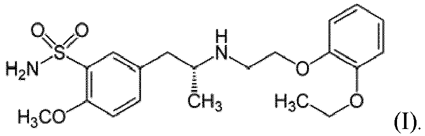

It is disclosed in EP 34432 and US 4,731,478. Tamsulosin is a potent pharmaceutically active agent and is typically administered in amounts of less than 1 mg per day, usually 0.4 mg.

Several medicaments comprising tamsulosin, specifically tamsulosin hydrochloride, are currently marketed. The first commercially available medicament comprising tamsulosin (Omnic, Flomax, marketed since 1993) were gelatin capsules filled with pellets comprising tamsulosin (0.4 mg strength).

A controlled release tablet formulation was introduced on the market in Europe (Omnic OCAS^{®}, Mapelor OCAS^{®}) also containing 0.4 mg of tamsulosin hydrochloride. The tablet is purported to have less or no food effect in comparison to the capsule formulation. This marketed tablet medicament uses the so called OCAS^{®} (oral-controlled absorption system) drug delivery system which enables gradual release of a drug as the tablet travels through the digestive tract, including the colon. The relevant patent covering the OCAS^{®} drug delivery is a patent family based on WO 94/06414 (EP 661045, US 6,436,441). In general, the OCAS^{®} comprises a drug, a hydrogel-forming polymer and a highly water soluble hydrophilic additive (a "hydrophilic base"), which is important in aiding rapid water penetration into the core. The concept is that the preparation absorbs water as it enters and passes through the upper digestive tract such that the water-swellable polymer undergoes substantially complete gelation (i.e. at least 70%). As the preparation continues down the digestive tract, its gelled surface continues to constantly erode, thereby maintaining a release of the drug even in the lower digestive tract, e.g., in the colon.

A preferred hydrogel-forming polymer according to US 6,436,441 is a poly(ethylene oxide), especially one having a molecular weight of not less than 2 million. Another proposal for the delivery of tamsulosin throughout the whole digestive tract from a tablet formulation was disclosed in WO 2007/131804. The composition comprises a) 0.1 to 1 weight % of tamsulosin, preferably tamsulosin hydrochloride; b) 40-80 weight % of a water-swellable matrix- forming composition comprising (i) a pH- sensitive swellable hydrophilic polymer, which is a cross-linked polyacrylic acid polymer and preferably is carbomer, and (ii) a pH-insensitive swellable hydrophilic polymer, which preferably is a linear polymer such as hydroxypropyl methylcellulose (HPMC), especially a high molecular weight HPMC; and c) optionally a water insoluble binder. While the tablets of WO 2007/131804 exhibit a good release profile of tamsulosin, the tablet design (composition, size, shape, etc.) again requires a careful balance of properties; and achieving sufficient release in the colon is often problematic for this kind of controlled release tablet.

Solifenacin is chemically described as 1-azabicyclo[2.2.2]oct-3-yl (1R)-1-phenyl-3,4-dihydro-1H-isoquinoline-2-carboxylate. It has the structure of formula (II):

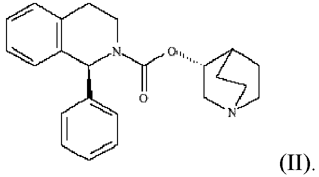

Solifenacin is a competitive muscarinic acetylcholine receptor antagonist belonging to the class of urinary antispasmodic. Solifenacin is marketed under the tradename Vesicare^{®} as a film-coated tablet that contains either 5 mg or 10 mg solifenacin succinate. The tablet is approved for the symptomatic treatment of urge incontinence and/or increased urinary frequency and urgency as may occur in patients with overactive bladder syndrome.

WO2010051996 teaches that antimuscarinic agents can be combined with alpha adrenoreceptors for treating lower urinary tract symptoms associated with beningn prostatic hyperplasia (LUTS/BPH). Both solifenacin and tamsulosin are specifically disclosed as examples of antimuscarinic agents and alpha adrenoreceptors.

A tablet relating to a pharmaceutical composition combining a modified release portion comprising tamsulosin and a polymer that forms a hydrogel with an immediate release portion comprising solifenacin is disclosed in WO2010090172. In this application is explained that when the commercial solifenacin formulation Vesicare^{®}, which contains lactose monohydrate, starch, hypromellose and magnesium stereate, is used; the dissolution rate of solifenacin is reduced. WO2010090172 teaches that if at least one hydrophilic substance from the group consisting of D-mannitol, maltose, polyethyleneglycol, and polyvinylpyrrolidone (PVP) is used in the solifenacin immediate release part, this problem is solved.

However, maltose is a reducing sugar which is known to give stability problems in compounds with primary and secondary amines, due to Maillard reactions. Tamsulosin has secondary amines which may react with maltose. On the other hand, PVP is known to have peroxides as impurities which may give stability problems.

Thus, in view of the prior art cited above, there is still a need for alternative pharmaceutical compositions comprising tamsulosin and solifenacin which are stable and suitable for use on a commercial scale.

### BRIEF DESCRIPTION OF THE PRESENT INVENTION

The present invention provides a wet granulation process for the preparation of the immediate release part of the multilayer tablet comprising:
- A controlled release part comprising tamsulosin; and
- An immediate release part comprising:
   a) Solifenacin succinate;
   b) A water insoluble diluent in an amount of 50 to 99% w/w relative to the total weight of the immediate release part of the tablet, wherein the insoluble diluent is a combination of 25% dicalcium phosphate and 75% microcrystalline cellulose.

The process comprising:
a. Combining solifenacin with a water insoluble diluent, wherein the insoluble diluent is a combination of 25 % dicalcium phosphate and 75% microcrystalline cellulose;
b. Granulating the resulting mixture with a granulation solvent;
c. Drying the resulting granulate;
d. Further mixing the obtained granulate with one or more further pharmaceutically acceptable excipients to form a further mixture.

Said pharmaceutical composition may be used as a medicament, particularly in the treatment of moderate to severe storage symptoms (urgency, increased micturition frequency) and voiding symptoms associated with benign prostatic hyperplasia (BPH).

### DETAILED DESCRIPTION OF THE PRESENT INVENTION

The present invention provides a wet granulation process for the preparation of the immediate release part of the multilayer tablet comprising:
- A controlled release part comprising tamsulosin; and
- An immediate release part comprising:
   a) Solifenacin succinate;
   b) A water insoluble diluent in an amount of 50 to 99% w/w relative to the total weight of the immediate release part of the tablet, wherein the insoluble diluent is a combination of 25% dicalcium phosphate and 75% microcrystalline cellulose.

The process comprising:
a. Combining solifenacin with a water insoluble diluent, wherein the insoluble diluent is a combination of 25 % dicalcium phosphate and 75% microcrystalline cellulose;
b. Granulating the resulting mixture with a granulation solvent;
c. Drying the resulting granulate;
d. Further mixing the obtained granulate with one or more further pharmaceutically acceptable excipients to form a further mixture.

Solvents suitable to perform the wet granulation are water or alcohols or mixtures thereof. Alcohols are preferred. When alcohols are used the drying process is faster. Furthermore, when alcohol is used the drug product contains less impurities. The preferred alcohol is ethanol. Most preferred alcohol is ethanol 96%.

The multi-layer tablet of the invention has a composition comprising:
- A controlled release part comprising tamsulosin; and
- An immediate release part comprising:
   a) Solifenacin succinate;
   b) A water insoluble diluent, in an amount of 50 to 99% w/w relative to the total weight of the immediate release part of the tablet.

A multilayer tablet is typically produced by compactation of different mixtures in the form of various layers in a single tablet. It generally consists in parallel distinct layers with two or more APIs optionally along with functional or non functional placebo layers. The non functional placebo layers may avoid interaction between different incompatible layers. More preferably the tablet of the invention is a bilayer tablet. Bilayer tablets are suitable for sequential and simultaneous release of two different APIs. In that case, one layer is immediate release and another layer is controlled release. A bilayer tablet according to the present invention contains a controlled release part comprising 0.4 to 1.6 mg of tamsulosin and an immediate release part comprising 1 to 30 mg of solifenacin and a diluent which is water insoluble. The preferred dosage strength of tamsulosin is 0.4 mg; the preferred dosage strengths of solifenacin are 6 and 10 mg. Presently preferred forms are bilayer tablets comprising 0.4/6 mg of tamsulosin and solifenacin respectively.

Each layer comprises different excipients, so as to give suitable properties for compression, lubrication, binding as is well known to one skilled in the art.

Diluents are fillers which are used to increase the bulk volume of a tablet or capsule. By combining a diluent with the active pharmaceutical ingredient, the final product is given adequate weight and size to assist in production and handling.

The term "water insoluble" as used herein means that the solubility in water of the diluent is lower than 0.05 g/100 ml water, measured at 20 °C at 1atm pressure.

In order to get the stabilization effect, the amount of diluent(s) ranges from 50 to 99% w/w relative to the total weight of the immediate release part of the tablet, preferably from 60 to 98% w/w, even more preferably from 70 to 97% w/w, most preferably from 80 to 95% w/w relative to the total weight of the immediate release part of the tablet.

Suitable examples of diluents to be used in accordance with the present invention include alkali metal inorganic salts, more preferably calcium or magnesium inorganic salts, such as calcium carbonate, magnesium carbonate or dicalcium phosphate. In a preferred embodiment dicalcium phosphate is used as a diluent. Dicalcium phosphate can be used in its anhydrous form or as a hydrate. Dicalciumphosphate has good compactation properties and good flow properties of the coarse grade material.

Another suitable example of diluent to be used in accordance with the invention is microcrystalline cellulose (MCC).

In a most preferred embodiment microcrystalline cellulose and dicalcium phosphate are used together as a diluents, for instance as in the commercial available Avicel DG which is a powder containing 75% of microcrystalline cellulose and 25% anhydrous dicalcium phosphate. When MCC and/or phosphate derivatives such as dicalcium phosphate are used, the stability of the bilayer tablet of the invention is improved.

The tablet composition of the present invention preferably contains at least one glidant in the immediate release part of the tablet with a specific surface area ≤400 m²/g.

A glidant is added in order to improve the flowability of the immediate release part of the multilayer tablet. Suitable glidants are magnesium stereate, magnesium silicate, starch, talc and silicon dioxide. Silicon dioxide is the most preferred glidant because it reduces van der Waals attractive forces between excipients and solifenacin resulting in an enhanced flowability. It also enhances the uniformity content of solifenacin in the powder blend. A range from 0.05 to 10% w/w of glidant is preferred relative to the total weight of immediate release part of the tablet, more preferred is a range from 0.1 to 5% w/w, even more preferred is a range from 0.2 to 2% w/w relative to the total weight of immediate release part of the tablet.

In order to avoid interactions with the release of tamsulosin, a rapid disintegration time of the immediate release part of the tablet is desired. To achieve this goal disintegrant can be added to the formulation of the immediate release part.

Disintegrants are agents added to tablet formulations to promote the breakup of the tablet (and capsule "slugs') into smaller fragments in an aqueous environment thereby increasing the available surface area and promoting a more rapid release of the drug substance.

Suitable examples of disintegrants to be used in accordance with the present invention include crospovidone, starch, sodium croscarmellose and mixtures thereof. In a preferred embodiment sodium croscarmellose is used as a disintegrant to avoid oxidation impurities. A range from 1 to 10% w/w of disintegrants is preferred, more preferred is a range from 2 to 5% relative to the total weight of the immediate release part of the tablet.

The immediate release part of the multilayer tablet according to the present invention may comprise other excipients as for example binders and lubricants.

Binders ensure that tablets and granules can be formed having the desired or required mechanical strength, and they give volume to low active dose tablets. Binders which are suitable for use in accordance with the present invention include povidone, hydroxypropyl methylcellulose, hydroxy propylcellulose, and sodium carboxyl methylcellulose.

The tablet composition of the invention may also contain a lubricant. Lubricants are generally used in order to reduce sliding friction. In particular, to decrease friction at the interface between a tablets surface and the die wall during ejection, and reduce wear on punches and dies. A range from 0.25 to 5% w/w of lubricants is preferred, more preferred is a range from 0.5 to 2.5% relative to the total weight of the immediate release part of the tablet.

Suitable lubricants to be used in accordance with the present invention include magnesium stearate, calcium stearate, stearic acid, glyceryl behenate, hydrogenated vegetable oil, and glycerine fumarate. Preferably, magnesium stereate is used as lubricant.

A preferred multilayer tablet according to the present invention is a tablet wherein the solifenacin immediate release part comprises:
a. A water insoluble diluent in an amount of 50 to 99% w/w relative to the total weight of the immediate release part of the tablet, preferably of 60 to 98% w/w, even more preferably of 70 to 97% w/w, most preferably of 80 to 95% w/w relative to the total weight of the immediate release part of the tablet; wherein the insoluble diluent is a combination of 25% dicalcium phosphate and 75% microcrystalline cellulose;
b. A glidant with a specific surface area ≤ 400 m²/g in an amount of 0.05 to 10% w/w relative to the weight of the immediate release part of the tablet;
c. A disintegrant in an amount of 1 to 10% w/w relative to the total weight of the immediate release part of the tablet.

The control release part according to the present invention, comprising tamsulosin, may comprise any of the excipients described above. Preferably the control release part of the bilayer tablet comprises a hydrogel forming polymer and at least one diluent having a water solubility of less than 0.2 g/ml at temperature of 20 °C. A hydrogel forming polymer is a polymer that upon contact with an aqueous medium it hydrates rapidly to form a gel layer. Examples of these polymers are polyethylenoxide (such as PEO 7.000.000), hydroxypropylmethylcellulose, or hydroxyethylcellulose. A preferred hydrogel forming polymer is PEO 7.000.000.

Diluents having a water solubility of less than 0.2 g/ml at temperature of 20 °C are preferably selected form the group of calcium carbonate, magnesium carbonate or dicalcium phosphate or Microcrystalline cellulose (MCC). Most preferably, MCC is used as a diluent.

The bilayer tablet of the present invention is stable and shows an in vitro dissolution profile wherein tamsulosin is released at least 20% within 4 hours, at least 45% within 8 hours and at least 80% after 20 hours when the composition is subjected to a dissolution study in 500 ml phosphate buffer (pH 6.8) using a USP apparatus II at 50 rpm at 37°C.

The term "stable" as used herein means that tablets comply with the purity and the dissolution specification when subjected to a 6 months stability study at the accelerated stability conditions of 40°C and 75% RH

In a preferred embodiment of the invention the solifenacin part of the tablet has a dissolution rate of 70% or more in 10 min, even more preferred 80% or more in 10 minutes, when the composition is subjected to a dissolution study in 900 ml water using a USP apparatus II at 50 rpm at 37°C.

The pharmaceutical compositions described herein can be made using conventional methods and equipment well-known in the art such as wet granulation, dry granulation or mixing. Wet granulation is preferred for the immediate release part of the multilayer tablet. The present invention further relates to a tablet composition as described hereinabove, comprising:
- A controlled release part comprising tamsulosin; and
- An immediate release part comprising:
   a) Solifenacin succinate;
   b) A water insoluble diluent in an amount of 50 to 99% w/w relative to the total weight of the immediate release part of the tablet, wherein the insoluble diluent is a combination of 25% dicalcium phosphate and 75% microcrystalline cellulose;
   wherein the immediate release part is prepared by a wet granulation process, which process comprises the steps of:
   a. Blending solifenacin with a water insoluble diluent; wherein the insoluble diluent is a combination of 25 % dicalcium phosphate and 75% microcrystalline cellulose;
   b. Granulating the resulting mixture with a granulation solvent;
   c. Drying the resulting granulate and sieving it if applicable;
   d. Further mixing the obtained granulate with one or more pharmaceutically acceptable excipients to form a further mixture.

Solvents suitable to perform the wet granulation are water or alcohols or mixtures thereof. Alcohols are preferred. When alcohols are used the drying process is faster. Furthermore, when alcohol is used the drug product contains less impurities. The preferred alcohol is ethanol. Most preferred alcohol is ethanol 96%.

Typically, the controlled release part according to the present invention is prepared by a fluid bed wet granulation process, which process comprises the steps of:
a. Dissolving tamsulosin in granulation liquid;
b. Spraying tamsulosin solution over diluent to granulate and afterwards drying the resulting granules;
c. Sieving of dry granules;
d. Blending extragranular excipients including an hydrogel-forming polymer.

In the multilayer tablets the interaction of the different APIs can result in stability problems. In order to avoid these problems, the particles of tamsulosin and/or solifenacin may be optionally coated. Another option is to coat the granules of solifenacin and/or tamsulosin obtained in the above processes. Furthermore, a non functional layer (layer without API) can be added between the tamsulosin and solifenacin layer to avoid the interactions of the two drugs. This non functional layer can comprise excipients such as sugars, for instance lactose, cellulose derivatives such as MCC or phosphates derivatives such as dicalcium phosphate, cellulose and phosphates derivatives are preferred.

The apparatus and method used in the step of forming the multilayer tablet are well known in the art. Bi-layered tablets may be prepared by laminating the modified release portion and the immediate release portion, or by compressing the modified release portion and subsequently compressing the immediate release portion; by eventually adding a drug-free layer between the modified release portion and the immediate release portion. Examples of a tabletting machine include a multilayered rotary tabletting machine. The multilayer tablet may be further coated with a film coat.

The tablet composition of the present invention is suitable for use as a medicament e.g for the treatment of moderate to severe storage symptoms (urgency, increased micturition frequency) and voiding symptoms associated with benign prostatic hyperplasia (BPH).

The following examples are intended to illustrate the scope of the present invention but not to limit it thereto.

### Preparation of the Immediate release part of the multilayer tablet

### Example 1

**Table 1 - Qualitative and quantitative formula examples 1**

| **Wet granulation in the presence of ethanol** | | |
|---|---|---|
| **Component/Function** | **mg** | **% immediate release layer** |
| Solifenacin succinate/API | 6.0 | 6.0% |
| Microcrystalline cellulose 75%: Dicalcium Phosphate Dihydrate 25%(Avicel DG)/Diluent | 89.3 | 89.3% |
| Na croscarmellose /Disintegrant | 3.0 | 3.0% |
| Silicon dioxide (Aerosil)/Glidant | 0.6 | 0.6% |
| Magnesium stearate/Lubricant | 1.0 | 1.0% |
| Iron oxide (red) | 0.1 | 0.1% |
| Ethanol 96% | | q.s. |
| *Total weight content* | *100.0* | *100.0%* |

The immediate release part of Example 1 was made according to the process depicted in the following scheme:

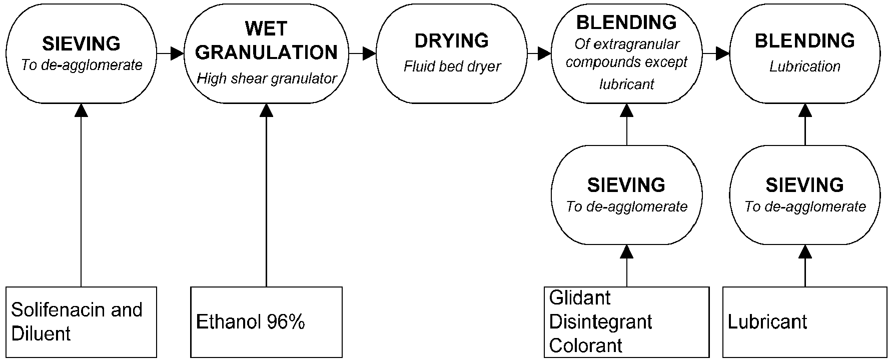

### Example 1a

**Table 2 - Qualitative and quantitative formula examples 1a**

| **Wet granulation in the presence of ethanol** | | |
|---|---|---|
| **Component/Function** | **mg** | **% immediate release layer** |
| Solifenacin succinate/API | 6.0 | 3.60% |
| Microcrystalline cellulose 75%: Dicalcium Phosphate Dihydrate 25%(Avicel DG)/Diluent | 152.82 | 91.73% |
| Na croscarmellose /Disintegrant | 5.0 | 3.00% |
| Silicon dioxide (Aerosil)/Glidant | 1.0 | 0.60% |
| Magnesium stearate/Lubricant | 1.66 | 1.00% |
| Iron oxide (red) | 0.12 | 0.07% |
| Ethanol 96% | | q.s. |
| *Total weight content* | *166.60* | *100.00%* |

### Preparation of the controlled release part of the multilayer tablet

### Example 2:

**Table 3 - Qualitative and quantitative formula example 2**

| **Components** | **mg/tablet** | **% control release layer** |
|---|---|---|
| **Intragranular** | | |
| Tamsulosin.HCl | 0.4 | 0.17 |
| Microcrystalline cellulose | 40.4 | 16.83 |

| **Extragranular** | | |
|---|---|---|
| PEO 7.000.000 | 198.0 | 82.5 |
| Magnesium stearate | 1.2 | 0.5 |

The controlled release part of Example 2 was made according to the process depicted in the following scheme:

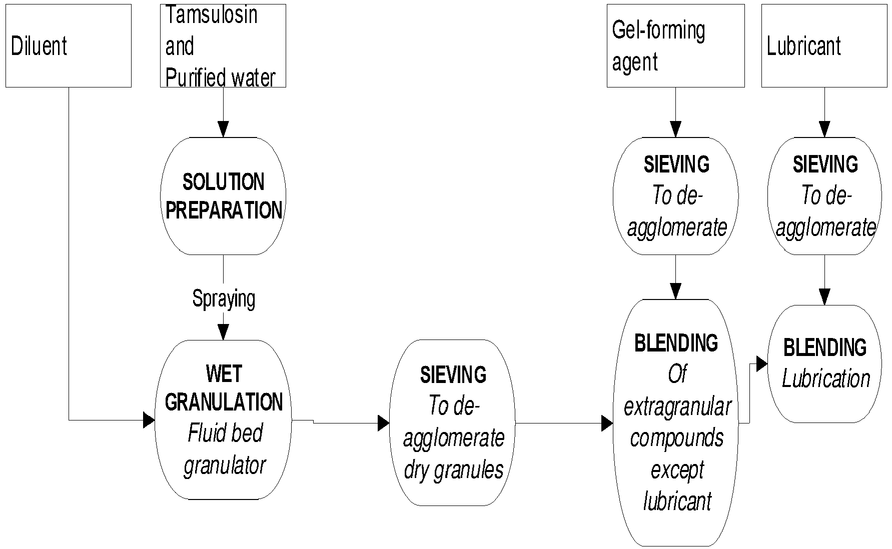

### Tableting the multilayer tablet

Using a multilayered rotary tableting machine, one part of the modified release portion (from example 2) and one part of the immediate release portion (from example 1) were formed into bilayered tablets, to obtain a pharmaceutical composition for oral administration of the present invention containing 0.4 mg of tamsulosin and 6 mg of solifenacin succinate.

For this purpose, tamsulosin is compressed, subsequently the dicalcium phosphate layer is compressed and finally the solifenacin layer is added.

## Claims

1. A wet granulation process for the preparation of the immediate release part of the multilayer tablet comprising:
• A controlled release part comprising tamsulosin; and
• An immediate release part comprising:
a) Solifenacin succinate;
b) A water insoluble diluent in an amount of 50 to 99% w/w relative to the total weight of the immediate release part of the tablet, wherein the insoluble diluent is a combination of 25% dicalcium phosphate and 75% microcrystalline cellulose.
The process comprising:
a. Combining solifenacin with a water insoluble diluent, wherein the insoluble diluent is a combination of 25 % dicalcium phosphate and 75% microcrystalline cellulose;
b. Granulating the resulting mixture with a granulation solvent;
c. Drying the resulting granulate;
d. Further mixing the obtained granulate with one or more further pharmaceutically acceptable excipients to form a further mixture.

2. The process according to claim 1 wherein alcohol is used as granulation solvent.

3. The process according to claim 2 wherein ethanol is used as granulation solvent.
